# EUROPEAN PATENT APPLICATION

(11) **EP 3 406 624 A1**
(43) Date of publication of application: **28.11.2018**
(21) Application number: 17172844.7
(22) Date of filing: 24.05.2017
(51) Int. Cl.: C07K 1/22, B01J 20/282

(54) **USE OF A NITROGEN-DOPED POROUS CARBON MATERIAL FOR ENRICHING PHOSPHORYLATED PROTEINS OR PEPTIDES**

(71) Applicant: University Of Amsterdam, 1012 WX Amsterdam (NL)
(72) Inventor: CAMENZULI, Michelle, 1098 XH Amsterdam (NL); KOOT, Stan, 1098 XH Amsterdam (NL); ROTHENBERG, Gadi, 2341 NV Oegstgeest (NL)
(74) Representative: De Vries & Metman

(57) **Abstract**

The present invention pertains to the use of a nitrogen-doped porous carbon material for the enrichment of phosphorylated peptides and/or proteins. The advantage of using a carbon-derived material is that it provides high surface area, high permeability containing both mesopores and macropores. Furthermore it retains polar and non-polar compounds and is extremely chemically stable. The key advantage of using a nitrogen-doped porous carbon material is the fact that it can be positively charged at low pH. This charge is crucial for the enrichment of phosphorylated proteins and peptides. The nitrogen-doped porous carbon material enriches all types of phosphorylated peptides; single, double, triple and quadruple phosphorylated. To our best knowledge this is the first material capable of achieving such a feat.

## Description

### Field of the invention

The present invention is directed to the use of a nitrogen-doped porous carbon material for enriching phosphorylated proteins and/or peptides in addition to a method for the enrichment of phosphorylated proteins and/or peptides.

### Background of the invention

Proteins are responsible for carrying out and regulating all of the processes that occur within our bodies; such as regulating our metabolism, cell-division and DNA replication. Some proteins are modified after their synthesis to carry out specialized functions. These modifications are known as "post-translational modifications" (PTMs). One of the most important PTMs is the addition or removal of phosphate groups from the protein (known as phosphorylation). Proteins that carry the phosphate PTM are known as phosphorylated proteins. These proteins regulate a large number of important cell-signalling cascades regulating a variety of processes important for the healthy functioning of our bodies. Errors in phosphorylation can distrupt these pathways and lead to the development of disease. In fact, variations in phosphorylation have been linked to prostrate cancer and diabetes among other diseases. Accordingly, studying phosphorylated proteins is of the upmost importance.

Studying phosphorylation requires the enrichment of phosphorylated proteins and peptides. Enrichment is essential because phosphorylated proteins are present in very low amounts compared to other proteins therefore they will not be detected without enrichment.

Several techniques are known for the enrichment of phosphorylated peptides/proteins. After enrichment it is possible to identify the compounds using techniques such as mass spectrometry or high performance liquid chromatography-mass spectrometry (HPLC-MS, or LC-MS). Existing techniques for enrichment are namely fractionation or separation, for instance using ion exchange or hydrophilic interaction (HILIC) chromatography resins. Alternatively other techniques are used namely immunoprecipitation, immobilized metal affinity chromatography (IMAC),metal oxide affinity typically with titanium oxide or zirconium dioxide, sequential elution from IMAC (SIMAC) and chemical derivatization. The most commonly used techniques by far are titanium dioxide (TiO₂) zirconium dioxide and immobilized metal affinity chromatography. These materials have been used in various ways; as beads, combined with other materials such as silica, extruded into fibers or made into monoliths to name a few, however the implementation of enrichment techniques falls into one of two workflows that is classified as either offline or online. In an offline workflow, enrichment is performed manually as a series of steps prior to analysis using LC-MS analysis as a distinct separate step. Online enrichment occurs as part of the LC-MS analysis via use of a trap column. The trap column contains the material for enrichment of phosphorylated proteins and/or peptides and connected to the injection valve of the LC-MS system. The idea being that the sample is enriched by the trap column prior to elution and separation using a separation column prior to entering the MS part of the system for further structural characterization.

Regardless of the form in which these materials are applied, they are unable to detect all of the sites in a protein where phosphorylation occurs. Furthermore they are unable to enrich both single and multiple phosphorylated peptides simultaneously. No existing material is able to provide a full picture of where protein phosphorylation occurs because they cannot efficiently enrich all types of phosphorylated peptides.

In the prior art also the use of some carbon materials has been proposed. For instance, the use of mesocrystalline tin dioxide nanorods on reduced graphene oxide sheets has been described and also the use of titanium dioxide (TiO₂) on magnetic graphene. The above-mentioned techniques are either tedious, complicated techniques that require a lot of experience from the people working with them or are not universal for all phosphorylated proteins or cannot distinguish between single and multiple phosphorylated proteins. None of these techniques provide a full picture of where phosphorylation occurs.

As a result, there is a demand for better tools to allow phosphorylated proteins to be studied in a reliable way, providing maximum information on phosphorylated proteins. Our invention provides a new method for enrichment of phosphorylated peptides and/or proteins. The invention described herein differs from other enrichment techniques due to the use of a nitrogen-doped carbon material that has demonstrated the ability to enrich both single and multiple phosphorylated peptides.

The nitrogen-doped porous carbon material used in this invention is known from the art but it was used for other purposes, for instance as a material for electrodes as described in US2012/0241691. The afore-mentioned patent application describes the use of nitrogen-doped porous carbon as electrode material in supercapacitors. The material is obtained by mixing melamine (1,3,5-triazine-2,4,6-triamine) with magnesium citrate, and heating the resultant mixture to 700ºC or more in an inert atmosphere, followed by cooling and washing with acid to remove magnesium oxide. Knowledge of nitrogen-doped porous carbon material was also disseminated in D. Eisenberg et al., A Simple Synthesis of an N-doped Carbon ORR Catalyst: Hierarchical Micro/Meso/Macro Porosity and Graphitic Shells, Chem. Eur. J. 2016, 22, 501-505, describes a nitrogen-doped porous carbon material comprising 5.7% atomic nitrogen.

### SUMMARY OF THE INVENTION

The present invention thus pertains to the use of a nitrogen-doped porous carbon material for enrichment of phosphorylated peptides and/or proteins. The advantage of using carbon is that it provides high surface area, high permeability, it contains both mesopores and macropores, it has shape selectivity, it retains polar and nonpolar compounds and is extremely chemically stable.

In one embodiment of the invention the nitrogen-doped porous carbon material is positively charged to retain phosphorylated proteins and/or peptides. The positive charge ensures the entrapment of the phosphorylated peptides and/or proteins.
In an embodiment of the invention the nitrogen-doped porous carbon material has the following properties:
- a nitrogen content in the range of 0.50 to 8.0 at% as determined via XPS (X-ray photoelectron spectroscopy),
- a specific surface area (N₂, BET, (Brunauer-Emmett-Teller)) of at least 1000 m²/g,
- a total pore volume as determined by N₂ adsorption of at least 0.80 ml/g,
- a N₂ adsorption pore size distribution which is such that at least 50% of the pore volume is in pores with a diameter of above 20 nm.

These properties ensure a high mass loading capacity allowing a large amount of protein to be loaded and enriched without losing protein due to breakthrough (that is, saturation).

In an embodiment of the invention the nitrogen-doped porous carbon material is used in combination with titanium dioxide for the enrichment of phosphorylated proteins and or peptides. To this end both the titanium dioxide and the nitrogen-doped porous carbon material may be packed together in a container such as a column or a pipette tip.

The invention is further directed to a process for enriching phosphorylated proteins and/or peptides wherein:
a. a protein and/or peptide containing sample interacts with a nitrogen-doped porous carbon material in acidic conditions, so that phosphorylated proteins and/or peptide present in the sample are retained,
b. releasing the phosphorylated proteins and/or peptide from the nitrogen-doped porous carbon material by eluting at basic conditions, obtaining enriched phosphorylated proteins and/or peptides.

The nitrogen-doped porous carbon material may be packed in any container of sutable size and material. Examples of suitable containers are a chromatographic column or a pipette tip. Said column may optimally be used for online analysis with LC-MS.

It was further found that the nitrogen-doped porous carbon material could be used for LC separation of the enriched phosphorylated proteins and/or peptides.

In offline enrichment, which is suitably performed by packing the nitrogen-doped carbon material in a pipette tip, the enriched phosphorylated proteins and/or peptides may subsequently be analyzed with LC-MS.

### BRIEF DESCRIPTION OF THE DRAWING

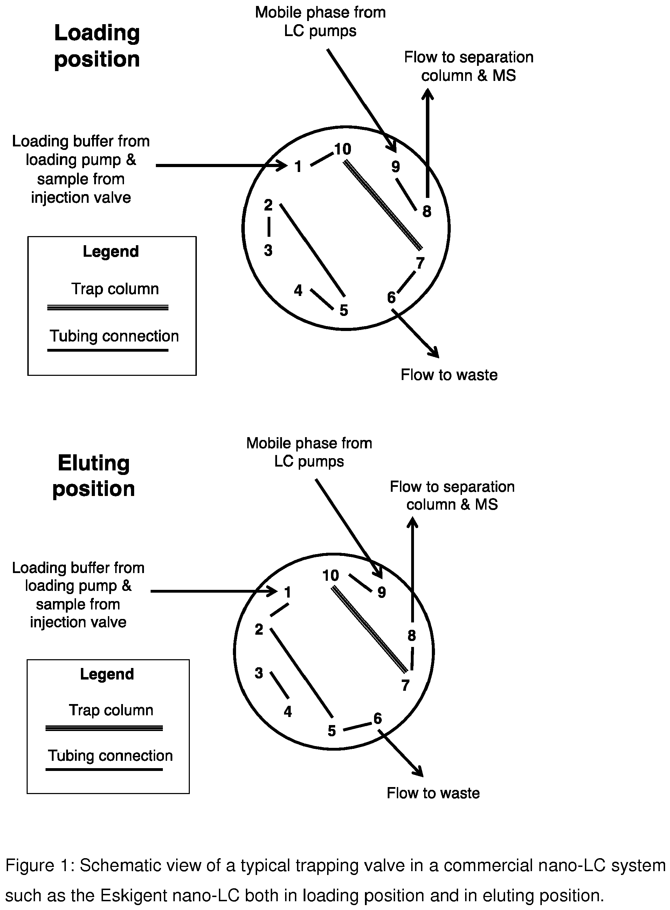

Fig. 1 depicts a schematic view of a typical trapping valve in a commercial nano-LC system both in loading position and in eluting position..

### DETAILED DESCRIPTION OF THE INVENTION

The various aspects of the present invention will be elucidated further below.

### Nitrogen-doped porous carbon material

As mentioned-above, the present invention pertains to the use of a nitrogen-doped porous carbon material for enrichment of phosphorylated peptides and/or proteins. This material is completely different from the materials so far used for enrichment. It is a nitrogen-doped carbon which has been synthesized in such a way that it can be positively charged at low pH. This charge is crucial for the enrichment of phosphorylated proteins and peptides (which carry negative charges). Due to its carbon nature, it is also capable of hydrophobic interactions that provide another mechanism for trapping phosphorylated peptides that is not used by alternative methods for enrichment, such as TiO₂.

The nitrogen-doped porous material is pre-equilibrated by washing in acidified solvent so that it becomes positively charged in order for the phosphorylated proteins and/or peptide present in the sample to be retained by the material.

Preferably the nitrogen-doped porous carbon material has the following properties:
- a nitrogen content in the range of 0.50 to 8.0 at% as determined via XPS (X- ray photoelectron spectroscopy)
- a specific surface area (N₂, BET) of at least 1000 m²/g,
- a total pore volume as determined by N₂ adsorption of at least 0.80 ml/g,
- a N₂ adsorption pore size distribution which is such that at least 50% of the pore volume is in pores with a diameter of above 20 nm.

These properties ensure a high mass loading capacity allowing a large amount of protein to be loaded and enriched without losing protein due to breakthrough (that is, saturation). More specifically, the nitrogen-doped porous carbon material used in in accordance with the present invention has a nitrogen content in the range of 0.50 to 8.0 at%. It may be preferred for the nitrogen content to be in the range of 1.0-8.0 at.%, in particular in the range of 2.0-8.0 at.%. A range of 4.0 to 8.0 at% may be particularly preferred, as it is believed that higher nitrogen contents leads to an increased trapping of the phosphorylated peptides or proteins.

The nitrogen content of the nitrogen-doped porous carbon material used in accordance with the present invention can be determined via X-Ray Photoelectron Spectroscopy (XPS) using a PHI VersaProbe II scanning XPS microprobe (Physical Instruments AG, Germany). Analysis is performed using a monochromatic Al Kα X-ray source of 24.8 W power with a beam size of 100 µm. The spherical capacitor analyser is set at 45° take-off angle with respect to the sample surface. The pass energy was 46.95 eV. Integration is performed and adjusted to remove the occasionally occurring Si 2p peaks (< 3%). Peaks are calibrated using the C1 s position. Curve fitting is performed using the XPSPeak 4.1 freeware, and assigned based on literature values. Reference is made to Chem. Eur. J. 2016, 22, 501-505, cited above.

The nitrogen can be present in different configurations in the nitrogen-doped porous carbon material used in the present invention, such as at the edges of graphitic plains (pyridinic nitrogen, N-pyr), inside graphitic planes (graphitic nitrogen, N-graph), in more oxidized environments (N-oxy). The different types of nitrogen can be determined using XPS as described above. Graphitic nitrogen is generally present in an amount of 35-80%, calculated on the total amount of atomic nitrogen, in particular in the range of 40-75%, more in particular in the range of 45-75%.Oxydized nitrogen is generally present in an amount of 3-25%, calculated on the total amount of atomic nitrogen, in particular in the range of 3-20%, more in particular in the range of 5-18%.Pyridinic nitrogen is generally present in an amount of 10-50%, calculated on the total amount of atomic nitrogen.

The nitrogen-doped porous carbon material has a surface area as determined by BET from the nitrogen adsorption isotherm of at least 1000 m²/g. In the context of the present invention the nitrogen adsorption isotherm can be measured on a Thermo Scientific Surfer instrument using N₂ at 77 K. The samples are dried in a vacuum (1·10⁻³ mbar) for 3 h at 200 °C prior to the measurement. Helium density is measured on a Micromeretics multi volume pycnometer 1305. Isotherms are analyzed by the ThermoFisher Advanced Data Processing 6.0 software. Specific surface areas and micropore volumes can be calculated using the BET2 and the Dubinin-Radushkevitch methods, respectively. Micropore volume is the pore volume present in pores with a diameter below 2 nm. Total pore volume is the pore volume determined at relative pressure P/Po = 0.99. Mesopore volume is total pore volume minus micropore volume.

A higher specific surface area is considered preferred in the present invention as this is believed to contribute to a higher loading capacity. It is therefore considered preferred for the nitrogen-doped porous carbon material to have a specific surface area of at least 1100 m²/g, in particular at least 1200 m²/g. As a maximum a value of 3000 m²/g may be mentioned. The nitrogen-doped porous carbon material used in the present invention has a total pore volume as determined by nitrogen adsorption of at least 0.80 ml/g. A larger pore volume may be preferred as this is believed to contribute to an improved mass transfer of larger peptides and/or proteins. This enables the peptides/proteins to interact more effectively with the material. Large pore volume is especially important for proteins. It is therefore preferable for the nitrogen-doped porous carbon material to have a nitrogen adsorption pore volume of at least 0.90 ml/g, in some embodiments at least 1.0 ml/g. As maximum, a value of 3.5 ml/g may be mentioned.

Preferably the nitrogen-doped porous carbon material has its pores in the range of between 10 nm and 40 nm. It is a particular feature of the nitrogen-doped porous carbon material used in the invention that it has at least 50% of the pore volume in pores with a diameter of above 20 nm (nitrogen adsorption pore size distribution). It has been found that a higher percentage of pores in this range positively influences the trapping of proteins.

It is preferred for the nitrogen-doped porous carbon material to have at most 90% of its nitrogen adsorption pore volume in pores with a diameter of above 20 nm, in particular between 60 and 80%. Additionally or alternatively, it is preferred for the nitrogen-doped porous carbon material to have at least 0.5 ml/g of its nitrogen adsorption pore volume in pores with a diameter of above 20 nm, in particularly at least 0.6 ml/g. Specifically preferred materials have at least 0.8 ml/g of their nitrogen adsorption pore volume in pores with a diameter of above 20 nm, in particularly at least 1.0 ml/g, or even higher.

### Possible workflows for enrichment of phosphorylated proteins and/or peptides

The nitrogen-doped porous carbon material may be used both for offline and online enrichment. The biological material to be enriched may be both protein and peptides. Thus the protein may be enriched as such, i.e. enrichment via a top down workflow, or as peptides, i.e. enrichment via a bottom up workflow.

Prior to the enrichment procedure described by this invention a sample, typically of biological origin, is first prepared as per the desired workflow. Typically this involves digestion into peptides followed by desalting, if a bottom-up workflow is used. Alternatively samples can be prepared for enrichment without digestion therefore the proteins remain intact; referred to as a top-down workflow.

The present invention is further directed to a process for enriching phosphorylated proteins and/or peptides wherein:
a. a protein and/or peptide-containing sample is contacted with a nitrogen-doped porous carbon material in acidic conditions, so that phosphorylated protein and /or peptide present in the sample is retained
b. releasing the phosphorylated proteins and/or peptide from the nitrogen-doped porous carbon material by eluting at basic conditions, obtaining enriched phosphorylated proteins and/or peptides.

In order to suitably contact the protein and/or peptide-containing sample with the nitrogen-doped porous carbon material, the material is packed in a container. Any container of suitable size and material may be used. Examples of a suitable container is a column or pipette tip.

As, mentioned-above, the process for enrichment can be performed via two different methods, referred to herein as 'offline' and 'online'. The main difference between offline and online enrichment is that online enrichment is done in tandem with LC-MS by means of a trapping column. Offline enrichment is performed by packing the nitrogen-doped porous carbon material into a pipette tip. In an online approach enrichment is done in an automated manner within the LC-MS system. This is accomplished by packing the nitrogen-doped porous carbon material into a trap column rather than a pipette tip as described above.

In step a of the above-described process, the peptides or proteins are suspended in acidified solvent and brought into contact with the nitrogen-doped porous carbon material. The nitrogen-doped porous material is pre-equilibrated by washing in acidified solvent so that is becomes positively charged in order for the phosphorylated proteins and/or peptide present in the sample to be retained by the material. Upon contact with the positively charged material, the charged peptides/proteins bind to the material via ionic interactions and hydrophobic interactions due to the hydrophobicity of the material and the peptides/proteins.

For offline enrichment the protein and/or peptide-doped sample may be contacted with the nitrogen-doped porous carbon material by packing the material into a pipette tip. To this end the pipette tip is blocked off at the narrow end. Prior to packing the material into the pipette tip, the material is washed firstly with a solution of 10% trifluroacetic acid (v/v) in LC-MS grade water followed by washing with acetonitrile. After washing a slurry is prepared by suspending the material in water or ethanol. Typically a concentration of 20-70, preferably 50 mg/mL is used. Subsequently the slurry is transferred to the pipette tip. This may be done in any suitable way known in the art. A suitable way is by means of centrifugal force. For instance, the slurry is transferred to the pipette tip which is inserted into an Eppendorf tube which has had a hole drilled into it's lid in such a manner which allows the closed Eppendorf tube to hold the pipette tip in place with the narrow end of the tip inside the tube. The Eppendorf tube with the pipette tip is placed into a centrifuge and spun for a certain amount of time. Typically it is spun at 100-3000 rpm, preferably about 2000 rpm for 10 to 60 seconds, preferably about 30 seconds. The centrifugal force pushes the slurry into the pipette tip, making it suitable for use for enrichment of phosphorylated peptides and proteins.
The pipette tip now packed with the nitrogen-doped porous carbon material (hereinafter referred to as 'spin column') is removed from the centrifuge then washed with acidified solvent using centrifugal force. Any solvent containing an organic acid that may bring the final pH of the solvent to 2 or below may be used. The solvent also contains water and optionally an organic solvent when necessary to solve the protein or peptide properly. A suitable solvent is 6% trifluoroacetic acid in 80% acetonitrile and 20% purified water (v/v). The conditions for centrifugation are for instance at 100-3000 rpm for 10 to 60 seconds, preferably about 2000 rpm for 30 to 90 seconds, preferably about 60 seconds. This step ensures that the nitrogen-doped porous carbon material is in acidic conditions and consequently is positively charged.

The protein and/or peptide-doped sample may also be contacted with the nitrogen-doped porous carbon material by packing the material into a column, hereinafter referred to as trap column. This is especially suitable when conducting the process in an online workflow.
The trap column may be prepared by packing a fused silica capillary that has been partially sealed by means of a silica porous frit, which has been prepared by reaction of potassium silicate. The packing and frit-making procedure is known in the art. The trap column containing the nitrogen-doped porous carbon material may be installed in place of the standard trap column of an LC-MS apparatus. The location of the trap column varies based on the particular LC-MS configuration supplied by the LC-MS manufacturer. Typically, the trap column is connected to a valve that is connected to an injection valve, through which the sample is collected from a vial within the LC-MS autosampler. The trap column is supplied with mobile phase or loading buffer (i.e. the acidified solvent). A suitable loading buffer is an aqueous solution of trifluroacetic acid (from 0.1 to 6% v/v in LC-MS grade water with 3 % v/v LC-MS grade acetonitrile).

Also the protein and/or peptide-doped sample is acidified prior to contacting it with the nitrogen-doped porous carbon material. To this end the protein/ peptide sample is suspended acidic solvent at a pH of 2 or below after previously being concentrated by evaporation. Any solvent containing an organic acid that may bring the final pH of the solvent to 2 or below may be used. The solvent also contains water and optionally an organic solvent when necessary to solve the protein or peptide properly. Formic acid or trifluoroacetic acid at a concentration of between 0.01 % and 0.1 % v/v in water is ideal for this purpose. That said, the acid may be added to a mixture of organic solvent and water if the sample is not soluble in water alone. A specifically suitable solvent is 6% trifluoroacetic acid in 60-80% acetonitrile and 40-20% purified water (v/v). For offline enrichment the sample (typically about 200 microliter) is added to the spin column and pushed into the material within the spin column using the centrifugal force. Usually between 1000 and 300 rpm, preferably about 2000 rpm for 30-90 seconds, preferably 60 seconds is used. This step is repeated until the entire sample has been loaded onto the column.
For online enrichment the sample for enrichment containing phosphorylated peptides and/or proteins within a matrix such as a solvent, may simply be injected via the autosampler onto the trap column. The solvent for dilution or dissolving the sample should be similar in composition to the loading buffer, that is, it should be acidic with a pH of 2.
Because the sample has been suspended in acidic solvent (pH 2 or below) only very acidic peptides/proteins, in particular phosphorylated peptides/proteins, will be negatively charged and therefore bind with the positively charged nitrogen-doped porous carbon material. In offline enrichment the unbound non-phosphorylated peptides/proteins will pass through the spin column and enter the bottom of the 1.5 mL Eppendorf tube, and may be discarded to waste or kept for analysis once the Eppendorf becomes too full for use and must be emptied for subsequent steps. Once the sample has been completely loaded onto the spin column, the spin column is washed using 200 µL of 0.1% trifluoroacetic acid (v/v) in purified water. This step is performed to remove any non-phosphorylated peptides/proteins that may have remained in the spin column.
In the inline enrichment the unbound non-phosphorylated peptides/proteins are passed through the trap column and are consequently discarded as waste.

Subsequently, peptides/proteins are released from the material for analysis by LC-MS. The release (known in the field as 'elution') occurs by passing a basic solution through the nitrogen-doped porous carbon material. The flow-through is collected, which contains the now enriched phosphorylated peptides/proteins.
The basic conditions required for the subsequent release of the phosphorylated proteins and/or peptides may be set by using sodium hydroxide solution, ammonium hydroxide solution or ammonium bicarbonate solution. The pH is optimally set in a range between 7.0 and 11, preferably between 7.5 and 9.0.

In offline processing, elution may be achieved by passing a basic solution through the spin column using a plastic syringe with the liquid passing through the spin column being collected in a new 1.5 mL Eppendorf tube. The enriched phosphorylated peptides/proteins will be contained within the liquid collected in the Eppendorf tube. For example, 5% ammonium hydroxide (v/v) in water or 0.1 % ammonium bicarbonate (v/v) in water may be used. The basic solution may also include a proportion of organic solvent to overpower any hydrophobic interactions between the bound peptides/proteins and carbon within the material, thus aiding elution of the bound peptides/proteins. For example 0.1 % ammonium bicarbonate in 55% acetonitrile and 45% water may be used for this purpose.

For online processing elution may be achieved by passing basic solution through the material via the LC pumps in the case of online enrichment. The basic solution is comprised of any LC-MS compatible base so long as a pH between 7 and 11 is achieved. Recommended basic solutions for releasing the bound phosphorylated peptides/proteins in the online enrichment workflow are 0.1 % (v/v) ammonium bicarbonate in LC-MS grade water as mobile phase A and 0.1 % ammonium bicarbonate in 45% LC-MS grade water and 55% LC-MS grade acetonitrile (v/v) as mobile phase B. In the online enrichment workflow a linear gradient of the aforementioned A and B mobile phases is mixed by the LC pump system to produce a linear gradient ranging in composition from 5% mobile phase B to 95% mobile phase B (v/v) with the remaining percentage being made up from mobile phase A. The time duration of the gradient depends on the complexity of the sample to be analyzed and can be set accordingly by the user. As the gradient comprising mobile phases A and B passes through the trap column containing the nitrogen-doped porous carbon material, the bound phosphorylated peptides/proteins are released from the trap column and can now be described as enriched.

By carefully setting the elution gradient, the nitrogen-doped porous carbon material may also be used for separating the phosphorylated proteins and/or peptides.

Preferably, the enriched phosphorylated proteins and/or peptides are subsequently analyzed with LC-MS but other means for analyzing the enriched phosphorylated proteins and/or peptides may be used such as immunoblotting techniques, UV or fluorescence spectrometry. If the nitrogen-doped porous carbon material is used for separating the phosphorylated proteins and/or peptides, the separated fractions may be directed to a mass spectrometer without first passing the separation column of the LC-MS equipment.

The enriched phosphorylated peptides/proteins obtained after elution in an offline workflow may be desalted using a C18 spin column. Said C18 spin column may be created in a similar manner as the nitrogen-doped porous carbon material but instead of making a slurry and using glass microfiber discs, discs are cut out from C18 Empore C₁₈ discs and inserted into a new pipette tip as described earlier. Acetonitrile (typically 50 microliter) is used to wash the C18 spin column using a centrifugal force. This is followed by a wash with 50 microliter of 0.1 % trifluoroacetic acid (v/v) in purified water using centrifugal force. The enriched phosphorylated peptides/proteins sample collected from the nitrogen-doped porous carbon material spin column, is loaded onto the C18 spin column using the centrifugal force. This step may be repeated as required to ensure that the enriched sample is loaded in its entirety onto the C18 spin column. The C18 spin column, now including the bound enriched sample, is then washed with 0.1 % trifluoroacetic acid (v/v) in Milli-Q purified using the centrifugal force. A solution of 0.1 % formic acid in 80% acetonitrile, 20 % LC-MS grade water (v/v) is used to elute the desalted, enriched phosphorylated peptide/proteins are eluted from the C18 material into the Eppendorf tube. This desalting procedure may be conducted to avoid ion suppression during subsequent LC-MS analysis, which would decrease the sensitivity of the MS. The enriched, desalted phosphorylated peptides may be evaporated to dryness at 30 °C in a vacuum centrifuge prior to further analysis. The dried enriched sample is dissolved in a minimum amount of an aqueous solution of trifluroacetic acid (from 0.1 to 6% in LC-MS grade water with 3% LC-MS grade acetonitrile v/v) for further analysis.

The enriched phosphorylated proteins and/or peptides obtained via the process according to the invention may be subjected to LC-MS for analysis. The LC-MS system comprises a trap column containing reversed phase LC material, such as C18, and a separation column that also contains reversed phase material such as C18. Other separation columns can be used such as ion exchange LC columns. As mentioned-above the nitrogen-doped porous carbon material can also be operated as a separation column. Therefore, by careful setting the elution gradient the trapping column may be used for both enriching the phosphorylated proteins and/or peptides and separating the phosphorylated proteins and/or peptides. Alternatively, a second nitrogen-doped porous carbon column may be used downstream of the trapping column, to act as a separation column. However we describe here the use of a C18 LC column for separation prior to MS. The reason we use reversed phase LC columns for separation prior to MS is because of the compatibility of reversed phase LC mobile phases with MS. For the LC separation under reversed phase conditions with a separation column containing 5 µm fully porous C18 silica-based particles, 0.1 % (v/v) ammonium bicarbonate in LC-MS grade water is used as mobile phase A and 0.1 % ammonium bicarbonate in 45% LC-MS grade water and 55% LC-MS grade acetonitrile (v/v) is used as mobile phase B. The term "mobile phase" refers to the liquid used in an LC system to pass through the column to generate the separation. The designation of A and B for the mobile phases follows the conventional within the chromatography field for reversed phase liquid chromatography whereby the aqueous solvent is designated as mobile phase A whereas the organic solvent is designated as mobile phase B. A linear gradient of the afore-mentioned A and B mobile phases is mixed by the LC pump system to produce a gradient ranging in composition from 5% mobile phase B to 95% mobile phase B (v/v) with the remaining percentage being made up from mobile phase A. The time duration of the gradient depends on the complexity of the sample to be analyzed and can be set accordingly by the user. The MS system hyphenated with LC should have the ability to perform MS/MS in order to properly identify the phosphorylated peptides and proteins via their fragmentation patterns in the resulting mass spectra. MS/MS analysis can be performed in a number of ways such as neutral loss scanning in positive ion mode, targeted MS in negative ion mode, Data dependent acquisition (DDA) or data independent acquisition (DIA). In our case we used DDA, fragmenting the top 30 most abundant ions using rolling collision energy.

When using an online workflow, the enrich enriched phosphorylated peptides/proteins, after being released from the trap column, the pass through the LC system tubing and enter the separation column. Within the separation column the phosphorylated peptides/proteins are separated via chromatography followed by further analysis with MS as described above for the offline workflow.

Regardless of whether an offline or online approach is taken as described above, the nitrogen-doped porous carbon material when used as directed has demonstrated the ability to enrich not only peptides with a single phosphate group but also those with double, triple and quadruple phosphate groups. In the prior art, typically using TiO₂ or TiO₂ - containing materials, show a bias towards peptides with a single phosphate group.
It was found that that the phosphorylation sites identified by the nitrogen-doped porous carbon material are complementary to those identified using TiO₂. Therefore, the process for enriching phosphorylated peptides/proteins can be used in combination with titanium dioxide. Since both for the nitrogen-doped porous carbon material and titanium dioxide the same solvents and eluents can be used, the two materials may even be packed in the same container, e.g. in one a column or a pipette tip.

The nitrogen-doped porous carbon material used according to the invention may be prepared by a method comprising the steps of:
- preparing an aqueous solution comprising N-doped organic acid as anion, alkaline earth metal cations, and alkali metal cations,
- precipitating a mixture of alkaline earth metal salt of a N-containing organic acid and alkali metal salt of a N-containing organic acid from the aqueous solution,
- subjecting the precipitate to a pyrolysis step at a temperature between 500ºC and 1200°C in an inert atmosphere,
- contacting the product of the pyrolysis step with an acidic solution to leach earth alkali metal components and alkaline metal components from the product of the pyrolysis step,
- optionally subjecting the leached product to a further heating step at a temperature of at least 800ºC in an inert atmosphere.

The first step in the process according to the invention is preparing an aqueous solution comprising nitrogen-containing organic acid as anion, alkaline earth metal cations, and alkali metal cations. It has been found that the use of a combination of alkaline earth metal cations and alkali metal cations makes it possible to tailor the nitrogen content of the material thus obtained. The solution can be prepared starting out from the respective salts of nitrogen-containing organic acid. However, it is also possible to prepare the solution in various other manners. In one embodiment, the solution is prepared by combining in an aqueous medium an N-containing organic acid, an inorganic alkaline earth metal salt, and an inorganic alkali metal salt. For example, one can start out with a solution of N-containing organic acid, followed by the addition of earth alkali metal salt and alkaline metal salt, added in combination or one after the other, in solid form or in dissolved form. The salt of the earth alkali metal salt and alkaline metal salt in this embodiment is generally an inorganic salt, preferably selected from carbonates, oxides, and hydroxides. To be able to obtain an aqueous solution it is required that the salts present therein have a solubility in water which is such that a reasonable concentration of the various components can be obtained. It is therefore preferred for the alkaline earth metal salt of the nitrogen-containing organic acid and the alkali metal salt of a nitrogen-containing organic acid to have a solubility in the solution medium of at least 0.05 mol/l, in particular at least 0.1 mol/l. It is within the scope of the skilled person to check the solubility of the materials selected.

The alkaline earth metals suitable for use in the invention include magnesium, calcium, strontium, and barium, with calcium and magnesium being preferred for reasons of costs and availability. Magnesium is considered particularly preferred as leaching of magnesium oxide particles from the carbon material after the heat treatment has been found easy to perform. The alkali metals suitable for use in the invention include lithium, sodium, potassium, rubidium, and cesium. Sodium and potassium are preferred for reasons of costs and availability.

The molar ratio between the alkaline earth metal and the alkali metal generally is in the range of 10:1 to 0.1:1, in particular in the range of 5:1 to 1:1. In general, the use of less alkali metal (and thus a higher ratio between the alkaline earth metal and the alkali metal) leads to a higher nitrogen content in the final product, which is associated with a higher ability to trap phosphorylated peptides/proteins.

In general, the nitrogen-containing organic acid comprises at least one nitrogen atom and 2-20 carbon atoms. The organic acid may be a carboxylic acid, but other acids such as sulphonic acids may also be used. The use of carboxylic acids is generally considered preferred, as the use of sulphonic acids will result in the addition of sulphur to the system, which may bring up HSE issues. Nitrogen-containing polycarboxylic acids may be preferred. It may be preferred for the compound to have the nitrogen as a secondary amine position, preferably at a tertiary amine position. Additionally or alternatively, it may be preferred for the compound to have at most 8 carbon atoms connected together, in particular at most 6, in some embodiments at most 4. Examples of suitable acids include nitrilotriacetic acid, iminodiacetic acid, and ethylenediamine tetra acetic acid.

The next step in the preparation of the nitrogen-containing porous carbon material is precipitating the mixture of alkaline earth metal salt of a nitrogen-containing organic acid and alkali metal salt of a nitrogen-containing organic acid from the aqueous solution. This can be done in various ways. One method is the addition of further compounds to the system to decrease the solubility of the salts in the aqueous medium. Suitable further compounds are in themselves miscible with water. Lower alcohols and ketones such as C1-C4 alcohols and C3-C6 ketones have been found suitable.
A further possibility would be to select the nature of the acid, the alkaline earth metal and the form in which it is added, and the alkali metal and the form in which it is added in such a manner that a precipitate is formed upon combination of the various components. A further possibility is of course the evaporation of water, which will result in the formation of a precipitate. Combinations of the various methods can also be applied.

The precipitate is isolated from the solution in manners known in the art, e.g., by filtration, optionally followed by washing. It is generally dried before being processed further. The precipitate is then subjected to a pyrolysis step at a temperature of between 500ºC and 1200°C in an inert atmosphere. In the pyrolysis step, the organic acid is carbonized, to form a nitrogen-doped carbon structure, while the alkaline-earth and alkaline metal ions form oxide particles and/or etch pores into the structure. The reaction is carried out in an inert atmosphere to prevent combustion and other reactions. Suitable inert atmospheres are known to the skilled person and include an argon, nitrogen, or helium atmospheres, at least 99.9% pure (no more than 0.1% O₂, and preferably even less).

The pyrolysis step is preferably carried out at a temperature between 600ºC and 1100°C, more preferably at least 700ºC, still more preferably between 800ºC and 1000°C. The pyrolysis reaction generally takes between 15 minutes and 12 hours, depending on the reaction temperature, with higher temperatures being associated with shorter reaction times. A reaction time of 15 minutes to 6 hours may be preferred, more preferably 30 mins to 5 hours, even more preferably 1-3 hours. The heating rates generally can be 1 to 20 °C/min, more preferably 5 to 10 °C/min.
The inert gas generally can be flowed at rates of 20 to 200 sccm, preferably at 80 to 150, for at least 30 minutes before the heat treatment, preferably for at least 90 minutes.

When the pyrolysis reaction has been completed, the reaction product is cooled to a temperature at which further processing is possible, generally between 5 and 95ºC. The product is then brought into contact with an acidic solution to leach earth alkali metal components and alkaline metal components from the product of the pyrolysis step. The acidic solution is generally an aqueous solution of an organic or inorganic acid. Suitable organic acids include C1-C6 mono, di, or tri-carboxylic acids, and inorganic acids such as HCl, sulphuric acid, nitric acid, and phosphoric acid. The concentration of the acidic solution is such that the pH of the solution is sufficiently low to affect adequate leaching. Suitable pH values are in the range of 0 to 5, more preferably 1 to 3.

If so desired, the product of the acid leaching step can be submitted to a further heating step at a temperature between 800ºC and 1200°C in an inert atmosphere. It may be preferred for the further heating step to carried out at a temperature between 900 and 1100ºC. For the atmosphere and reaction time reference is made to the description of the previous pyrolysis treatment. The aim of this step is to promote graphitization, to remove surface groups that may have been introduced by the acid (e.g. sulphonate groups due to sulphuric acid washing), and to decrease the amount of nitrogen incorporated in the material.

It is noted that various elements of the procedure described for synthesizing the nitrogen-doped porous carbon material, including but not limited to preferred ranges for the various parameters, can be combined unless they are mutually exclusive.

The invention will be elucidated by the following Examples, without being limited thereto or thereby.

### EXAMPLES

### Synthesis of nitrogen-doped porous carbon materials

Materials were synthesized using a modified version of the method described in D. Eisenberg et al., A Simple Synthesis of an N-doped Carbon ORR Catalyst: Hierarchical Micro/Meso/Macro Porosity and Graphitic Shells, Chem. Eur. J. 2016, 22, 501-505. All chemicals were purchased from commercial sources and used as received.

Nitrilotriacetic acid (N(CH₂COOH)₃, 22.937 g, 0.120 mol, 99%, Alfa Aesar A11936) was added to 300 mL of de-ionized (DI) water at 85 °C in a 2000 mL beaker, followed by addition of basic magnesium carbonate ((MgCO₃)₄Mg(OH)₂, 11.657 g, 0.120 mol Mg; 99%, <0.02% Fe, Strem 93-1220), to give a 1:1 molar ratio of nitrilotriacetate (NTA³⁻) to Mg²⁺. The solution became clear yellow within 1 minute. Different amounts of potassium carbonate (K₂CO₃, Alfa Aesar) were added (0 to 6 g), resulting in the formation of a solution of an alkaline earth metal salt of a N-containing organic acid and a alkali metal salt of a N-containing organic acid. The pH was measured after cooling to room temperature.

To precipitate the solid reproducibly, 1500 mL ethanol were flowed continuously over 5-6 minutes from a burette, with stirring and without heating. This produced off-white goo, with different degrees of graininess (more with higher pH values). After addition of ethanol, the solution was chilled in an ice bath for 1-2 hours, and the precipitate was collected and vacuum dried at 40 °C for 48 hours. The precipitate was ground by mortar and pestle to a fine white powder. Two salts were recrystallized from different solution compositions, and their structure and composition was determined by single crystal X-ray diffraction to determine the content of [MgNH(CH₂COO)₃(H2O)₃]·CH₃CH₂OH and [KMgN(CH₂COO)₃(H₂O)₂]·H₂O.

The materials described above were subjected to a heat and acid treatment as described below. Of each material, 20-25 g was transferred into a quartz boat, and loaded into a quartz tube. The tube was loaded into a tube furnace inside a fume hood, and heated to 900°C at 10 °C/min, held at 900 °C for 1 hr, and let cool naturally. Argon gas (99.999%) was flowed through the tube at a flow rate of 100 sccm for 90 mins before heating started, and continuously during pyrolysis.

The carbons were removed from the tube and added directly into 500 mL of 0.5 citric acid. Direct addition to the acid prevents the formation of potassium-containing products on the tubes, which may heat up and ignite the carbon upon exposure to ambient atmosphere. Each carbon was stirred in the acid for over 10 hours, and then vacuum filtered, washed with 5 L of deionised water, and dried for several hours at 120°C. Then each carbon was loaded again into a tube furnace and heated to 1000 °C at 5 °C/min, held for 1 hr, and let cool naturally. The reaction heating reaction again took place under argon, as described above. The total yield of each process is 5-15% (lower with higher KMgNTA fraction). All carbons were black and fine.

The properties of the materials thus obtained are presented in Table 1 below.

**Table 1:**

| Ex | K₂CO₃ added during preparation (wt.%) | pH | at% N | %N-pyr of total N | % N-graph of total N | % N-oxy of total N | BET2 SSA (m²/g) | total PV ml/g |
|---|---|---|---|---|---|---|---|---|
| 1 | 0 | 3.7 | 5.7 | 33.4 | 51.4 | 15.2 | 1831 | 3.1 |
| 2 | 2 | 5.0 | 3.7 | 31.1 | 55.8 | 13.1 | 1809 | 2.5 |
| 3 | 4 | 5.7 | 1.3 | 20.8 | 71.7 | 7.56 | 1508 | 1.3 |
| 4 | 6 | 6.1 | 1.3 | 16.5 | 70.0 | 13.6 | 1250 | 1.0 |
| 5 | 8 | 7.8 | 1.2 | 24.1 | 63.5 | 12.4 | 2080 | 1.4 |

### Use of nitrogen-doped porous carbon material for enrichment

### EXAMPLE 2: OFF-LINE WORKFLOW

### Example 2A: Sample preparation: digestion

In this example we used a bottom-up proteomics workflow, that is, the protein was digested into peptides prior to enrichment and analysis using LC-MS. The bottom-up workflow is the most common workflow currently used for identification and quantification of proteins in the field of proteomics. Digestion can be done in a number of ways but ultimately the same result is obtained: protein is digested into peptides. For this experiment an Immobilized-Enzyme Reactor (IMER) was used as published in: Wouters, B., Dapic, I., Valkenburg, T.S.E., Wouters, S., Niezen, L., Eeltink, S., Corthals, G.L., Schoenmakers, P.J. A cyclic-olefin-copolymer microfluidic immobilized-enzyme reactor for rapid digestion of proteins from dried blood spots (2017) Journal of Chromatography A, 1491, pp. 36-42.

In this technique, the proteins are unfolded in-solution and pumped through the reactor, which consist out of a capillary with a high concentration of trypsin immobilized onto the stationary phase. As the protein passes through the reactor, it comes into contact with the trypsin immobilized onto the surface of column material and is consequently digested into peptides.
More specifically, a solution of alpha-casein (Sigma Aldrich) was prepared at a concentration of 1.0 mg/mL in 50 mM hydrochloric acid (Acros Organics), 20 mM calcium chloride (Sigma Aldrich) buffer solution and inserted into a syringe. The syringe was equipped to the ANTEC Leyden syringe pump (KD Scientific) which was introduced the alpha-casein solution to the reactor at 1.0 µL/min and the digested proteins were collected in a 2 mL Eppendorf tube (Eppendorf).

### Example 2B: Sample preparation:desalting

After digestion via the IMER, the alpha-casein sample was desalted using an Empore solid phase extraction C18-SD 4 mm/1 mL cartridge (Sigma-Aldrich). The solid phase extraction cartridge was wetted using 1 mL of acetonitrile on 2000 rpm for 60 seconds in the model 5804R centrifuge (Eppendorf). Next, the equilibration step was done using 1 mL of 0.1 % (v/v) trifluoroacetic acid (sigma-Aldrich) in water purified using an Arium 611 UV water purification system (Satorius). Equilibration was performed using the centrifuge at 2000 rpm for 60 seconds. Sample (1 mL) was loaded onto the solid phase extraction cartridge using the centrifuge at 2000 rpm for 60 seconds twice to ensure that the entire sample was loaded onto the cartridge. Contamination was washed off with two washing steps using 0.1 % trifluoroacetic acid (v/v) in purified water at 2000 rpm for 60 seconds in the centrifuge. Elution was done using 1 mL of 6% trifluoroacetic acid in 80% LC-MS grade acetonitrile (Biosolve), 20% purified water (v/v) at 2000 rpm for 60 seconds in the centrifuge. The sample was stored at -20 °C prior to enrichment.

### Example 2C:Packing for off-line enrichment:

The nitrogen-doped porous carbon material was packed into a 200 microliter pipette tip which has been blocked off at the narrow end by cutting 3 glass microfiber discs and pushing them into the narrow end of the pipette tip. Prior to packing the material into the pipette tip, the material was washed firstly with a solution of 10% trifluroacetic acid (v/v) in LC-MS grade water followed by washing with acetonitrile. After washing the slurry was prepared by suspending the material in water or ethanol at a concentration of 50 mg/mL. To pack the pipette tip, 100 µL of the slurry was transferred to the pipette tip which was inserted into a 1.5 mL Eppendorf tube which had a hole drilled into it's lid in such a manner which allows the closed Eppendorf tube to hold the pipette tip in place with the narrow end of the tip inside the tube. The Eppendorf tube with the pipette tip is placed into a centrifuge and spun at 2000 rpm for 30 seconds. The application of centrifugal force pushed the slurry into the pipette tip, making it suitable for use for enrichment of phosphorylated peptides and proteins. The pipette tip packed with the nitrogen-doped porous carbon material (herein referred to as 'spin column') was removed from the centrifuge and washed using 200 microliter of 6% trifluoroacetic acid in 80% acetonitrile and 20% purified water (v/v) using the centrifuge at 2000 rpm for 60 seconds. This step was conducted to ensure that the nitrogen-doped porous carbon material was in acidic conditions and consequently is positively charged.

### Example 2D: Sample loading

The sample obtained in Example 2B was suspended in 6% trifluoroacetic acid in 60-80% acetonitrile and 40-20% purified water (v/v) after previously being concentrated by evaporation. The sample (200 microliter) was added to the spin column as prepared in Example 2C and pushed into the material within the spin column using the centrifuge at 2000 rpm for 60 seconds. This step is repeated until the entire sample has been loaded onto the column.

### Example 2E: Elution of phosphorylated peptide

Elution is achieved by passing a basic solution comprising 5% ammonium hydroxide (v/v) in water or 0.1 % ammonium bicarbonate (v/v) in water may be used through the spin column using a plastic syringe with the liquid passing through the spin column being collected in a new 1.5 mL Eppendorf tube. The enriched phosphorylated peptides/proteins will are contained within the liquid collected in the Eppendorf tube.

### Example 2F: desalting of eluted phosphorylated peptide

The enriched phosphorylated peptides/proteins obtained after elution according to Example 2E were desalted using a C18 spin column. Said C18 spin column was created in a similar manner as the nitrogen-doped porous carbon material but instead of making a slurry and using glass microfiber discs, discs were cut out from C18 Empore C₁₈ discs and inserted into a new pipette tip as described earlier. Acetonitrile (50 microliter) was used to wash the C18 spin column using a centrifuge at 5000 rpm for 60 seconds. This was followed by a wash with 50 µL of 0.1 % trifluoroacetic acid (v/v) in purified water using the centrifuge at 5000 rpm for 60 seconds. The enriched phosphorylated peptides/proteins sample collected from our nitrogen-doped porous carbon material spin column was loaded onto the C18 spin column using the centrifuge at 5000 rpm for 120 seconds. This step is repeated as required to ensure that the enriched sample is loaded in its entirety onto the C18 spin column. The C18 spin column, now including the bound enriched sample, was then washed with 0.1 % trifluoroacetic acid (v/v) in Milli-Q purified water at 5000 rpm for 60 seconds using the centrifuge. The C18 spin column was removed from the Eppendorf tube and the Eppendorf tube was discarded. A new 1.5 mL Eppendorf tube is prepared by drilling a hole inside the lid so the that the C18 spin column sits inside the Eppendorf tube as described earlier. A solution of 0.1 % formic acid in 80% acetonitrile, 20 % LC-MS grade water (v/v) was pushed through the C18 spin column using a plastic syringe. During this step the bound enriched phosphorylated peptide/proteins are eluted from the C18 material into the Eppendorf tube.

### EXAMPLE 3: ONLINE WORKFLOW

Example 3A: Sample preparation: digestion was done in accordance with procedure described in Example 2A.

Example 3B: Sample preparation: desalting was done in accordance with the procedure described in Example 2B.

Example 3Ca: Column preparation
A fused silica capillary (Polymicro Technologies) was cut at a length of 25 cm with the diamond capillary column cutter (Sigma-Aldrich) for a flat cut. A solution of 25% (v/v) of formamide (Next Advance) in purified water was prepared and mixed by vortex with 100 µL of Kasil (Next Advance). A glass microfiber filter (Sigma-Aldrich) was laid onto a petri dish and 2 microL of the kasil/formamide mixture was added to make a wet spot. The cut fused silica capillary was cleaned with analytical grade ethanol (Sigma-Aldrich) and pierced five times through the wet spot by twisting the capillary. The capillary, with the frit-end down was put into a round bottom 2 mL Eppendorf tube and placed in a heating block for baking overnight at 85 °C to harden the frit.

Example 3Cb: Packing the trap column with nitrogen-doped porous carbon material A slurry of the nitrogen-doped porous carbon material was prepared by suspending 4 mg of the material in 200 microliter of analytical grade ethanol in a 2 mL Eppendorf tube. The slurry was thoroughly mixed using a vortex. A magnetic stir bar was added and the Eppendorf tube containing the slurry was put inside a typical capillary column packing pressure cell which was made in-house. The lid of the pressure cell was closed. The fritted column was put inside the fitting of the pressure cell till 1 mm from the bottom of the Eppendorf tube, with the frit pointed upwards and closed hand tight. The magnetic stirrer was turned on and nitrogen gas entered the packing cell at a pressure of 1000 psi. The nitrogen gas pushed the slurry from the Eppendorf tubing into the capillary, filling the fritted column with slurry. When the packed bed reached the desired length (5 cm) the nitrogen gas flow was stopped. After 15 minutes passed to allow the pressure inside the cell to reduce back to atmospheric conditions, the packed trap column was removed. A suitable connection was made to connect the packed trap column to the M-Class LC (Waters Corporation) and a flow of 100% LC-MS grade methanol (Biosolve) at 1.0 microliter/min was applied for 1 hour to wet and consolidate the column. After an hour the pressure was slowly decreased to zero and the column was disconnected from the LC. Stainless steel connections were added to the column to connect it with the LC-MS for online enrichment, separation and MS analysis.

### Example 3D: Sample loading and separation

Prior to loading the desalted alpha-casein digest sample as prepared according to Example 2. was evaporated to dryness using a vaccum centrifuge (GeneVac MiVac DNA concentrator, SP Scientific) set at 30°C for 30 min. The dried sample was dissolved in 25 microliter of 6% trifluoroacetic acid in 3% LC-MS grade acetonitrile, 97% LC-MS grade water (v/v) which was transferred to a standard, plastic HPLC 2 mL sample vial fitted with a low volume well.

The sample was loaded to the trap column as prepared in Example 3Cb. The trap column replaced the standard C18 trap column of the Eskigent Nano-LC (Sciex). In Figure 1 a schematic view is given of the trapping valve used for the online connection to the LC system. Both the loading position depicted and the eluting position of the valve. Herein buffer is loaded from a bufferpump and sample is loaded via injection valve **1.** The trapping valve comprises tubings with connections **1** to **10** to provide for the possibilities to switch to the various outlets. The injection valve is connected to the trap column via connection **10.** The outlet of the trap column may be switched to connect to the separation column and MS or to the waste outlet **6.** In the loading position the trap column is connected to the waste outlet **6,** so that unphosphorylated proteins and/or peptides move through the column and leave via the waste outlet. In the eluting position the trap column is connected to the sepeartion column and MS via outlet **8.**

The trap column was equilibrated with loading buffer: 6% trifluoroacetic acid in 80% LC-MS grade acetonitrile, 20% LC-MS grade water (v/v) at a flow rate of 2 microliter/min.

Downstream of the injection system and the trap column a C18 separation column was installed. The C18 separation column was packed using the same procedure as described for the trap column. The specifications of the separation column were: 75 µm inner diameter, bed length of 10 cm, packed with 5 µm fully porous C18 silica particles with a pore size of 20 nm (formerly Michrom BioResources Inc. now Bruker). The separation column was equilibrated with over 10 column volumes of mobile phase A: 5% (v/v) ammonium hydroxide (Sigma-Aldrich) in LC-MS grade water (Biosolve), and mobile phase B: 5% ammonium hydroxide in 45% LC-MS grade acetonitrile and 55% LC-MS grade water (v/v) and a ratio of 40% A, 60% B at a flow rate of 300 nL/min.

To begin the online enrichment procedure, a 10 µL injection of the alpha-casein desalted digestion was performed in an automated manner by the Eskigent nano-LC system (Sciex). Loading of the sample onto the trap column was done in acidic conditions using the loading buffer, after which, the valve was switched so that the trap column was in line with the separation column and mobile phase A and B (basic conditions). The elution gradient was automatically started at a flow rate of 300 nL/min from 60% B to 99% B in 50 min. After which, the wash step began for 10 min on 99% B. The gradient went back to 60% B in 2 min for an equilibration in preparation for the next analysis. Enriched phosphorylated peptides were released from the trap column as the basic mobile phases passed through the trap column towards the separation column.

### EXAMPLE 4:ANALYSIS WITH MS

The Eskigent Nano-LC was connected to a 5600+ QTOF MS (Sciex). The LC-MS was operated using the Analyst TF 1.7 software provided and the nanospray III electrospray (ESI) source from Sciex. The MS was set in positive mode, MS/MS was set in high sensitivity mode using an accumulation time of 250 ms and a range from 200 to 1800 *m*/*z*. It was an Intensity Dependent Acquisition (IDA) experiment, otherwise known as Data Dependent Acquisition (DDA) with the following criteria: charge state 2+ to 4+, intensity threshold of 100 cps, mass tolerance of 50 mDa, maximum number of candidate ions to monitor per cycle were 30 and dynamic exclusion of 15 s after 1 occurrence was applied. Accumulation time of 250 ms and a range of 400 to 1250 *m*/*z* settings were used for TOF-MS. A 2.2 kV was applied to the ESI spray with a curtain gas of 30 psi and a nebulizer gas of 20 psi, the heater interface temperature was 150 °C. MS data acquisition occurred during the entire LC separation.

### Example 5 Data processing and interpretation

The raw data was exported from the MS computer and was pre-processed using Protein Pilot software (version 5.0.1, Sciex). A database was created in the FASTA file format for the Bos Taurus species via downloading the protein primary sequences from Uniprot (http://www.uniprot.org/). The enzyme used for digestion, that is trypsin, was specified. Other parameters were set as follows:
- Sample type: Identification
- Cys alkylation: none
- Digestion: trypsin
- Instrument: TripleTOF 5600
- Special factors: Phosphorylation emphasis
- Species: Bos Taurus
- Detected protein threshold: > 0.05 (10%)
- Search effort: Thorough ID
- Run false discovery analysis was checked

After the data was processed using ProteinPilot, the software was used to convert the processed data to a .mgf file format which was exported for further data processing using SearchGUI (version 3.2.11, CompOmics, http://compomics.github.io/projects/searchgui.html). The parameters used for dataprocessing with SearchGUI were was follows:
- Fixed modifications: carbamidomethylation of C, Oxidation of M
- Variable modifications: phosphorylation of S, phosphorylation of T, phosphorylation of Y
- Digestion: Enzyme
- Enzyme: Trypsin
- Specificity: specific
- Max missed cleavages: 2
- Fragment Ion types: b and y
- Precursor m/z tolerance: 10.0 ppm
- Fragment m/z tolerance: 0.5 Da
- Precursor charge: 2-4
- Isotopes: 0-1
- Algorithms used for searching: X! Tandem, MS Amanda, MS-GF+, OMASSA, Comet, Tide, Andromeda

The processed data was visualized in PeptideShaker (version 1.16.3,CompOmics, http://compomics.github.io/projects/peptide-shaker.html). The indicators taken into consideration to judge the quality of the protein identifications were the sequence coverage and spectrum counts. Indicators considered for judging the quality of the peptide (for phosphorylated and non-phosphorylated peptides) were the number of validated peptide spectral matches (PSM), D-score, and the precursor m/z error. Identified phosphorylated peptides were further confirmed by manual inspection of the fragmentation pattern of the mass spectra.

For alpha-casein, which has two forms: alpha-casein S1 and alpha-casein-S2 using the nitrogen-doped porous carbon material permits the identification of different phosphorylation sites than what was found with TiO₂ for our example with alpha-casein (TABLE 1A and TABLE 1 B).

**TABLE 1A phosphorylated sites found for Alpha casein S1**

| Amino acid | Literature* | TiO₂ | Nitrogen-doped carbon |
|---|---|---|---|
| S | 56 | 56 | |
| D | - | | |
| S | 61 | 61 | 61 |
| S | 63 | 63 | 63 |
| T | - | 64 | 64 |
| S | 79 | | |
| S | 81 | | |
| S | 82 | 82 | |
| S | 83 | 83 | |
| S | 90 | 90 | |
| Y | - | | |
| Y | - | | |
| Y | - | | |
| S | 130 | 130 | 130 |

| | | | |
|---|---|---|---|
| * Phosphorylation sites from literature were obtained from the UniProt database and are a combination of the results of numerous studies | | | |

**TABLE 1B phosphorylated sites found for Alpha casein S2**

| Amino acid | Literature* | TiO₂ | Nitrogen-doped carbon |
|---|---|---|---|
| S | 23 | | 23 |
| S | 24 | | 24 |
| S | 25 | | 25 |
| S | - | | 31 |
| S | 46 | | - |
| S | 71 | | |
| S | 72 | | |
| S | 73 | | |
| S | 76 | | |
| T | 87 | 87 | |
| S | - | | 137 |
| S | - | | 144 |
| T | - | | 145 |
| T | 153 | 153 | |
| S | 158 | | 146 |
| S | - | | 158 |
| T | | | 159 |

| | | | |
|---|---|---|---|
| * Phosphorylation sites from literature were obtained from the UniProt database and are a combination of the results of numerous studies | | | |

The results show that 38% of compounds found were single phosphorylations, 16% were double phosphorylations, 3 % were triple phosphorylations, and 43% were quadrupole phosphorylations. With Titanium oxide enrichment 94% compounds found were single phosphorlylations, 6% were double phosphorylations, 3 % were triple phosphorylations.

These experiments show that with the process according to the invention it is possible to retain and enrich both single and multiple phosphorylated peptides, whereas TiO₂ has a preference to retain mostly single and IMAC for multiple phosphorylations.

The phosphorylation sites identified by the nitrogen-doped porous carbon material have shown to be complementary to those identified using TiO₂.

## Claims

1. Use of a nitrogen-doped porous carbon material for enriching phosphorylated proteins and/or peptides.

2. Use according to claim 1, wherein the nitrogen-doped porous carbon material is positively charged to retain phosphorylated proteins and/or peptides.

3. Use according to claim 1 or 2, wherein the nitrogen-doped porous carbon material has the following properties:
- a nitrogen content in the range of 0.50 to 8.0 at% as determined via XPS
- a specific surface area (N₂, BET) of at least 1000 m²/g,
- a total pore volume as determined by N₂ adsorption of at least 0.80 ml/g,
- a N₂ adsorption pore size distribution which is such that at least 50% of the pore volume is in pores with a diameter of above 20 nm.

4. Use according to claim 1, 2 or 3, wherein the nitrogen-doped porous carbon material is packed in a container such as a pipette tip or a column.

5. Use according to any one of the preceding claims wherein the nitrogen-doped porous carbon material for online enrichment of phosphorylated proteins and/or peptides.

6. Use according to any of the preceding claims 1-4 wherein the nitrogen-doped porous carbon material for offline enrichment of phosphorylated proteins and/or peptides.

7. Use according to any one of the preceding claims wherein the nitrogen-doped porous carbon material is used in combination with titanium dioxide.

8. Use according to any one of the preceding claims wherein the nitrogen-doped porous carbon material is used for Liquid Chromatography (LC) separations.

9. Process for enriching phosphorylated proteins and/or peptides wherein:
a. a protein and/or peptide-doped sample is contacted with a nitrogen-doped porous carbon material in acidic conditions, so that phosphorylated protein and /or peptide present in the sample is retained
b. releasing the phosphorylated proteins and/or peptide from the nitrogen-doped porous carbon material by eluting at basic conditions, obtaining enriched phosphorylated proteins and/or peptides.

10. Process according to claim 9, wherein the phosphorylated protein and/or peptide are retained by adsorption, absorption, hydrophobic interactions and/or ionic interactions.

11. Process according to claim 9 or 10, wherein the nitrogen-doped porous carbon material is packed in a container.

12. Process according to claim 11, wherein in addition to nitrogen-doped porous carbon material also titanium dioxide is packed in the container.

13. Process according to claim 11 or 12 wherein the container is a column.

14. Process according to claim 11 or 12, wherein the container is a pipette tip.

15. Process according to any one of claims 8 to 13, wherein the enriched phophorylated proteins and/or peptides are subsequently analyzed with liquid chromatography/mass spectroscopy (LC-MS).
